# EUROPÄISCHE PATENTANMELDUNG

(11) **EP 3 124 052 A1**
(43) Veröffentlichungstag der Anmeldung: **01.02.2017**
(21) Anmeldenummer: 15179205.8
(22) Anmeldetag: 30.07.2015
(51) Int. Cl.: A61K 49/10, A61K 38/08

(54) **VERFAHREN ZUR DIAGNOSE VON KARZINOMEN MITTELS IRGD UND MAGNETRESONANZTOMOGRAPHIE (MRT)**

(71) Anmelder: Piiper, Albrecht, 69121 Heidelberg (DE)
(72) Erfinder: PIIPER, Albrecht, 69121 Heidelberg (DE); SCHMITHALS, Christian, 60528 Frankfurt (DE); VOGL, Thomas J., 60322 Frankfurt (DE)
(74) Vertreter: Simandi, Claus

(57) **Zusammenfassung**

Die vorliegende Erfindung betrifft ein bildgebendes Diagnoseverfahren, umfassend ein Kontrastmittel samt einem Kontrastverstärker in einer Magnetresonanztomographie (MRT) gestützten Diagnose von Karzinomerkrankungen bzw. Karzinomen, insbesondere von hepatozellulärem Karzinom (HCC). Das Kontrastmittel betrifft bevorzugt eine Gadoliniumverbindung, vorzugsweise Gd-DTPA mit iRGD als Kontrastverstärker zur verbesserten Bildgebung in der MRT von Karzinomen, insbesondere HCC. Weiterhin beschreibt die Erfindung ein Verfahren zur Risikostratifizierung von Patienten und Probanden mittels vorgenannter Diagnose.

SEQUENCE LISTING

<110>  Piiper, Albrecht

<120>  Verfahren zur Diagnose von Karzinomen mittels iRGD und
       Magnetresonanztomographie (MRT)

<130>  API196-02

<140>  not yet known
<141>  2015-07-30

<160>  4

<170>  PatentIn version 3.3

<210>  1
<211>  9
<212>  PRT
<213>  Artificial

<220>
<223>  Tumorhoming-Peptide

<400>  1

Cys Arg Gly Asp Lys Gly Pro Asp Cys
1               5

<210>  2
<211>  9
<212>  PRT
<213>  Artificial

<220>
<223>  Tumorhoming-Peptide

<400>  2

Cys Arg Gly Asp Arg Gly Pro Asp Cys
1               5

<210>  3
<211>  9
<212>  PRT
<213>  Artificial

<220>
<223>  Tumorhoming-Peptide

<400>  3

Cys Arg Gly Asp Lys Gly Pro Glu Cys
1               5

<210>  4
<211>  9
<212>  PRT
<213>  Artificial

<220>
<223>  Tumorhoming-Peptide

<400>  4

Cys Arg Asp Gly Arg Gly Pro Glu Cys
1               5

## Beschreibung

Die vorliegende Erfindung betrifft ein bildgebendes Diagnoseverfahren, umfassend ein Kontrastmittel samt einem Kontrastverstärker in einer Magnetresonanztomographie (MRT) gestützten Diagnose von Karzinomerkrankungen bzw. Karzinomen, insbesondere von hepatozellulärem Karzinom (HCC). Das Kontrastmittel betrifft bevorzugt eine Gadoliniumverbindung, vorzugsweise Gd-DTPA mit iRGD als Kontrastverstärker zur verbesserten Bildgebung in der MRT von Karzinomen, insbesondere HCC. Weiterhin beschreibt die Erfindung ein Verfahren zur Risikostratifizierung von Patienten und Probanden mittels vorgenannter Diagnose.

Trotz der Fortschritte in den letzten Jahren ist die Therapie von soliden Malignomen, zu denen auch das hepatozelluläre Karzinom (HCC) gehört, in fortgeschrittenen Stadien nach wie vor unbefriedigend. Das HCC ist weltweit das fünfhäufigste Malignom und, aufgrund der erst späten Diagnose und dann fehlender erfolgversprechender Therapieoptionen, die dritthäufigste Ursache krebsbedingter Mortalität. Das HCC entwickelt sich in der Regel auf der Basis einer Leberzirrhose, die durch eine chronische Hepatitis entsteht. Wichtige Ursachen für eine Leberzirrhose sind Hepatitis B- und Hepatitis C-Virusinfektionen, Aflatoxin und Alkoholabusus. Kurative Therapieansätze des HCC sind die Lebertransplantation oder Resektion. Während die Transplantation in den BCLC-Stadien 0, A und ggf. B durchgeführt werden kann, ist die Tumorresektion nur im Stadium 0 sinnvoll. Die verbleibenden, höher gradigen Tumorstadien können ausschließlich durch lokalablative Verfahren (LITT, TACE) oder systemische Therapie palliativ (Chemotherapie mit Sorafenib) behandelt werden. Die frühe Diagnosestellung ist daher für eine erfolgreiche Therapie für solide Malignome ausschlaggebend.

Eine Verbesserung der Überwachung von Patienten mit hohem Risiko für die Entwicklung eines HCCs, d. h. Patienten mit Leberzirrhose, sowie der Diagnostik verdächtiger Knoten sind von zentraler Bedeutung für eine deutliche Verbesserung der Prognose von HCC Patienten. Die zuverlässigste bildgebende HCC-Diagnostik ermöglicht die dynamische Kontrast-Kernspin-Tomographie (Kontrast-MRT) sowie die Kontrast-Computertomographie. HCCs zeigen bei diesen Untersuchungen aufgrund der Versorgung über die A. hepatica ein charakteristisches arterielles An- und venöses Abstromverhalten, welches durch die niedermolekularen Kontrastmittel genutzt wird (Magnevist®, Primovist®). Kleine HCCs weisen jedoch selten die typischen radiologischen Kriterien auf und sind eher hypovaskulär aufgrund der bereits reduzierten Versorgung über die Portalvene und der noch nicht ausgebildeten arteriellen Hypervaskularisierung. Somit bleibt die klinisch äußerst bedeutsame Unterscheidung zwischen kleinen HCCs sowie Regenerat- und dysplastischen Knoten in der zirrhotischen Leber weiterhin schwierig.

Die Magnetresonanztomographie (MRT) ist ein bildgebendes Verfahren, das vor allem in der medizinischen Diagnostik zur Darstellung von Struktur und Funktion der Gewebe und Organe im Körper eingesetzt wird. Die Magnetresonanztomographie basiert auf sehr starken Magnetfeldern sowie elektromagnetischen Wechselfeldern im Radiofrequenzbereich, mit denen bestimmte Atomkerne (meistens die Wasserstoffkerne/Protonen) im Körper resonant angeregt werden, die dann im Empfängerstromkreis elektrische Signale induzieren. Kontrastmittel dienen dabei zur Verbesserung der Darstellung von Strukturen und Funktionen von Geweben/Organen im MRT. Bei der Magnetresonanztomografie kommen als Kontrastmittel vorrangig Gadolinium-Chelate zum Einsatz, die wegen der paramagnetischen Eigenschaft des Gadoliniumatoms zu einer Verkürzung der Relaxationszeiten (T1 und T2) in der Nähe des Kontrastmittels und damit zu einer helleren (signalreicheren) Darstellung von Strukturen führen.

Ausgehend von diesem Stand der Technik ist es daher die Aufgabe der Erfindung, neue Möglichkeiten zur Diagnose, Differentialdiagnose, Prognose und insbesondere zur Früherkennung von Karzinomen, insbesondere des hepatozellulären Karzinoms (HCC) sowie zur Risikostratifizierung solcher Karzinomerkrankungen zur Verfügung zu stellen.

Dabei soll vor allem die Technik der Magnetresonanztomographie in der Diagnose eines Karzinoms verbessert werden. Des Weiteren soll die vorliegende Erfindung ein neuartiges MRT - Kontrastmittel anbieten, welches den derzeit für die Darstellung des Karzinoms im MRT zur Verfügung stehenden Kontrastmittel an Spezifität und Sensitivität übertrifft. Damit ist eine frühere Erkennung auch kleiner Karzinome sowie Metastasen möglich, die bisher im Stand der Technik unerkannt bleiben.

Gelöst wird die oben gestellte Aufgabe durch die Verwendung von iRGD als Kontrastverstärker in einem Verfahren zur Diagnose und/oder Risikostratifizierung von Karzinomerkrankungen bzw. Karzinomen, umfassend eine Lanthanoidverbindung als Kontrastmittel, wobei eine MRT an einem Patienten oder Probanden durchgeführt wird. iRGD erlaubt vorteilhaft die verbesserte Aufnahme und spezifische Anreicherung eines Kontrastmittels in einem Karzinom, insbesondere von Lanthanoidverbindungen als Kontrastmittel.

Daher betrifft die Erfindung ein bildgebendes Verfahren zur Diagnose und / oder Risikostratifizierung von Karzinomerkrankungen, wobei mittels
a.) eines Kontrastmittels enthaltend eine freie Lanthanoidverbindung, und
b.) eines Kontrastverstärkers iRGD,
c.) eine Magnetresonanztomographie an einem Patienten/Probanden erfolgt.

Ferner betrifft die Erfindung ein Kontrastmittel enthaltend a.) ein freies Lanthanoid und b.) ein Kontrastverstärker iRGD zur Verwendung in einem bildgebenden Verfahren zur Diagnose und / oder Risikostratifizierung von Karzinomerkrankungen, wobei eine Magnetresonanztomographie an einem Patienten/Probanden erfolgt.

In einer weiteren bevorzugten Ausführungsform der Erfindung erfolgt die Magnetresonanztomographie mehrfach, wobei vorzugsweise in einer ersten Magnetresonanztomographie das
i.) Kontrastmittel enthaltend eine freie Lanthanoidverbindung ohne einem Kontrastverstärker iRGD verabreicht/appliziert wird oder
i.') ein Kontrastverstärker iRGD ohne ein Kontrastmittel enthaltend eine freie Lanthanoidverbindung verabreicht/appliziert wird und in einer zeitversetzten,
ii.) zweiten Magnetresonanztomographie das Kontrastmittel enthaltend eine freie Lanthanoidverbindung mit einem Kontrastverstärker iRGD verabreicht/appliziert wird.

Dies erlaubt eine Kontrolle des erzielten Effektes zur Verbesserung der Bildgebung durch den Kontrastverstärker als auch eine Standardisierung bzw. Normalisierung. Die zweite MRT kann vorzugsweise innerhalb von 10 min. bis 6 h, insbesondere 1 bis 24 h, insbesondere innerhalb mehrerer Tage, insbesondere innerhalb von 4 bis 24 h nach dem ersten MRT erfolgen.

Insbesondere kann eine entsprechende Injektionslösung verabreicht werden, wobei eine erste Injektionslösung kein iRGD enthält, jedoch ein Kontrastmittel enthaltend eine freie Lanthanoidverbindung und eine zweite ansonsten gleiche Injektionslösung zusätzlich iRGD aufweist.

Insbesondere kann eine entsprechende Injektionslösung verabreicht werden, wobei eine erste Injektionslösung kein Kontrastmittel enthaltend eine freie Lanthanoidverbindung enthält, jedoch iRGD und eine zweite ansonsten gleiche Injektionslösung zusätzlich ein Kontrastmittel enthaltend eine freie Lanthanoidverbindung aufweist.

Daher betrifft die Erfindung ebenfalls ein bildgebendes Verfahren zur Diagnose und / oder Risikostratifizierung von Karzinomerkrankungen, dadurch gekennzeichnet, dass mittels a.) eines Kontrastmittels enthaltend eine freie Lanthanoidverbindung oder a'.) ein Kontrastverstärker iRGD, b.) eine erste Magnetresonanztomographie an einem Patienten/Probanden erfolgt und zeitversetzt
c.) ein Kontrastmittel enthaltend eine freie Lanthanoidverbindung, und
d.) ein Kontrastverstärker iRGD,
e.) eine zweite Magnetresonanztomographie an einem Patienten/Probanden erfolgt.

Nutzbar im Kontext der vorliegenden Erfindung sind Kontrastmittel enthaltend einen paramagnetischen Stoff, wie Elemente aus den Übergangsmetallen, Lanthanoide und den Aktiniden, die, soweit gewünscht, kovalent oder nicht-kovalent an Komplexbildner (Chelatoren) oder Aminosäure-haltigen Makromoleküle gebunden sein können. Insbesondere bevorzugt werden Elemente ausgesucht aus der Gruppe beinhaltend Gd(III), Mn(II), Cu(II), Cr(III), Fe(II), Fe(III), Co(II), Er(II), Ni(II), Eu(III) and Dy(III). Besonders bevorzugte Elemente sind Gd(III), Mn(II), Cu(II), Fe(II), Fe(III), Eu(III) and Dy(III), insbesondere Mn(II) und Gd(III).

In einer weiteren sehr bevorzugten Ausführungsform umfasst das Kontrastmittel enthaltend einen paramagnetischen Stoff eine Verbindung eines Elements ausgewählt aus der Gruppe der Lanthanoide. Besonders bevorzugt ist, daß die Lanthanoidverbindung eine Gadoliniumverbindung ist. Vorzugsweise enthält das Kontrastmittel einen paramagnetischen Stoff, insbesondere eine komplexierte/chelatierte Lanthanoidverbindung. Bevorzugte Chelatoren der vorliegenden Erfindung umfassen: Acetylaceton (acac), Ethylendiamin (en), 2-(2-Aminoethylamino)ethanol (AEEA), Diethylentriamin (dien), Imino-diacetat (ida), Triethylentetramin (trien), Triaminotriethylamin, Nitrilotriacetat (nta), Ethylendiaminotriacetat (ted), Ethylendiamintetraacetat (edta), Diethylentriaminpentaacetat (DTPA) 1,4,7,10-Tetraazacyclododecan-1,4,7,10-tetraacetat (DOTA), Oxalat (ox), Tartrat (tart), Citrat (cit), Dimethylglyoxim (dmg), 8-Hydroxychinolin, 2,2'-Bipyridin (bpy) and 1,10-Phenanthrolin (phen).

Weiterhin betrifft die vorliegende Erfindung in einer nächsten bevorzugten Ausführungsform ein Kontrastmittel enthaltend einen paramagnetischen Stoff, insbesondere eine Lanthanoidverbindung, wobei die Gadoliniumverbindung ein Komplex aus Gadolinium und einem Chelator ist, insbesondere wobei der Chelator ausgesucht ist aus der Gruppe bestehend aus Diethylentriaminpentaessigsäure (DTPA) und 1,4,7,10-Tetraazacyclododecan- 1,4,7,10-tetraessigsäure (DOTA). Vorzugsweise ist die Gadoliniumverbindung Gd-DTPA, oder Derivate dieser Verbindung, wie beispielsweise Gd-EOB-DTPA (Primovist®) .

Als "Chelator" im Kontext der vorliegenden Erfindung soll ein Ligand mit mehr als einem freien Elektronenpaar verstanden werden, der mindestens zwei Koordinationsstellen (Bindungsstellen) eines Zentralatoms, hier insbesondere eines Gadoliniumatoms, einnehmen kann. Liganden und Zentralatom sind über koordinative Bindungen verknüpft. Das bedeutet, das bindende Elektronenpaar wird allein vom Liganden bereitgestellt. Der Gesamtkomplex von Zentralatom und Ligand wird als "Chelatkomplex" bezeichnet.

Wesentlich ist im Rahmen der hier beschriebenen Erfindung, dass das Kontrastmittel enthaltend einen paramagnetischen Stoff, vorzugsweise die Gadoliniumverbindung frei und ungebunden vorliegt, insbesondere nicht in einem Nanopartikel inkorporiert ist. Daher ist erfindungsgemäß eine freie Lanthanoidverbindung als Kontrastmittel besonders bevorzugt.

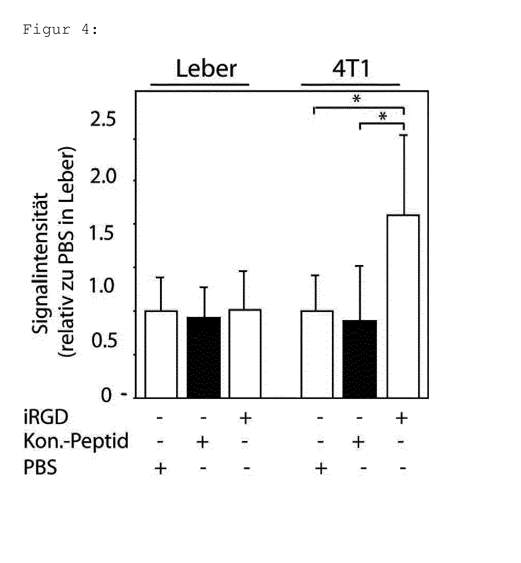

WO 2012/113733 A1 beschreibt ein Kontrastmittel ausschließlich auf der Basis eines Nanopartikels, wobei das Kontrastmittel als auch der Kontrastverstärker an einem Nanopartikel gebunden sind. Dies hat sich jedoch als nicht zielführend erwiesen (Waralee Watcharin, 2015), da die Injektion von Gd-DTPAhaltigen Humanserumalbumin (HSA)-Nanopartikeln eine negative Kontrastierung der HCCs in den Lebern mit HCC (Figur 4, Waralee Watcharin (supra)) tatsächlich bewirken. Es wurde gefunden, dass die HSA-Nanopartikel sehr rasch von den Makrophagen aufgenommen werden (Figur 7, Waralee Watcharin (supra)), so dass die Nanopartikel in der Leber verbleiben, da die Makrophagen-Dichte in der Leber höher ist als im Malignom. Zwecks einer guten Diagnose von Karzinomen, insbesondere HCC, sowie insbesondere eine hohe Auflösung der Bilder in der MRT zu ermöglichen, ist es bevorzugt, die Kontrastmittel der vorliegenden Erfindung in Verbindung mit vorzugsweise einem 3-Tesla-Tomographen in der MRT einzusetzen.

Es sind Karzinome erfindungsgemäß bevorzugt, die mit einer Störung der Barrierefunktion der Gefäße und vermindertem lymphatischen Abfluss in der pathologisch veränderten Region in Zusammenhang stehen. Dies trifft auf die meisten soliden Malignome zu, insbesondere das Mamma-Karzinom, Kolon-Karzinom, Pankreaskarziom, Magenkarzinom, Ovarial-Karzinom, Gallenwegs-Karzinom, Prostata-Karzinom, Zervix-Karzinom, Glioblastom, Bronchial-Karzinom, Pankreaskarzinom, Prostata-Karzinom, Nierenzellkarzinom, Blasenkarzinom, Hirntumore, Sarkome, hepato-zelluläres Karzinom oder auch Tumormetastasen dieser Karzinome davon.

Jedoch ist das hepato-zelluläre Karzinom (HCC) besonders bevorzugt.

In einer besonders bevorzugten Ausführungsform enthält das erfindungsgemäße Kontrastmittel weiterhin ein Tumorhoming-Peptid, insbesondere iRGD vorzugsweise mit den Sequenzen CRGDKGPDC (SEQ ID No.1), CRGDRGPDC (SEQ ID No.2), CRGDKGPEC (SEQ ID No.3) und CRDGRGPEC (SEQ ID No.4).

Kürzlich ist in unterschiedlichen Tumormodellen gezeigt worden, dass ein intravenös appliziertes Tumorhoming Peptid (iRGD) die Penetration von nieder- und hochmolekularen Substanzen selektiv im Tumorstroma in verschiedenen Tumormodellen verbessert (Sugahara et al, 2009; 2010). iRGD besteht aus einem RGD-Motiv, welches an Integrin aᵥ, einem bevorzugt im Endothel von Tumorgefäßen exprimierten Integrin (Ruoslahti, 2002) und einem CendR-Motiv, welches an Neuropilin-1 bindet. Die Bindung von CendR an Neuropilin-1 ist für den erhöhten tumorselektiven Penetrationseffekt erforderlich.

In der Dissertation (http://thesis.library.caltech.edu/ 7084/45/NG_SHEUNGCHEETHOMAS_2012_CH5.pdf) wird ebenfalls mittels Gd-DPTA und iRGD nachgewiesen, dass Tumorgefäße eine leicht erhöhte Permeabilität aufweisen. Es wird jedoch festgestellt, dass diese gefundene Permeabilität nicht hinreichend geeignet für eine MRT gestützte Diagnose ist.

Weiterhin beschreibt WO 2011/005540 A1 die therapeutische Anwendung mittels iRGD zur verbesserten Penetration von Krebsmitteln in Krebszellen.

In einer weiteren Ausführungsform kann das erfindungsgemäße Kontrastmittel zudem Mittel enthalten, die eine Erhöhung des Blutdrucks des zu diagnostizierenden Patienten / Probanden bewirken. So konnte nachgewiesen werden, daß eine Steigerung des Blutdrucks zu einer Verstärkung des so genannten EPR Effekts führt. Dies ermöglicht eine verbesserte Kontrastierung und somit eine spezifischere und sensitivere Diagnose. Mithin ist es besonders bevorzugt, daß das erfindungsgemäße Kontrastmittel Angiotensin II enthält. In einer weiteren Ausführung der Erfindung kann das Kontrastmittel ein physiologisch verträgliches wässriges Medium enthalten.

In einem weiteren Aspekt wird die Aufgabe der vorliegenden Erfindung durch ein Verfahren zur Diagnose und / oder Risikostratifizierung von Karzinomen, insbesondere hepatozellulärem Karzinom gelöst, umfassend, Verabreichen eines erfindungsgemäßen Kontrastmittels samt Kontrastverstärker an einen Patienten / Probanden und eine anschließende MRT Untersuchung des Patienten / Probanden. Dabei wird die MRT Untersuchung bevorzugt mittels eines 3-Tesla Tomographen vorgenommen.

Die vorliegende Erfindung kann allgemein zur Bildaufnahme mittels eines MRT bei einem Patienten / Probanden verwendet werden. Der Bildaufnahme-Vorgang wird dabei folgendermaßen durchgeführt: Zunächst wird einem Patienten eine ausreichende Menge des erfindungsgemäßen Kontrastmittels samt Kontrastverstärker verabreicht, um anschließend einen Scan des Patienten mittels Magnetresonanztomographie vorzunehmen. Ein invasiver Eingriff ist nicht erforderlich. Somit werden Bilder der inneren Strukturen des Patienten die von Interesse sind, insbesondere der erkrankten Gewebe, inkl. Karzinom, aufgenommen. Das erfindungsgemäße Kontrastmittel samt Kontrastverstärker ist zur Visualisierung von Gewebe, insbesondere Lebergewebe besonders hilfreich. Es kann zur Aufnahme von Bildern jede andere Region des Patienten / Probanden verwendet werden.

Die Verabreichung des erfindungsgemäßen Kontrastmittels samt Kontrastverstärker kann in jeder dem Fachmann bestens bekannter Art und Weise vorgenommen werden. Dies beinhaltet insbesondere die intravenöse, parenterale, intrakardiale (Herzinjektion), orale, rektale usw. Verabreichung von verschiedenen Formulierungen des Kontrastmittels. Die notwendige Dosis des Kontrastmittels wird entsprechend dem Alter, der Größe und des Gewichts des Patienten, sowie der zu Untersuchenden Körperregion variiert.

In einer bevorzugten Ausführungsform wird jedoch das erfindungsgemäße Kontrastmittel, vorzugsweise Gd-DPTA in einer Dosis von 45 - 65 µmol/kg, vorzugsweise 57 µmol/kg Körpergewicht appliziert. Die Applikation erfolgt vorzugsweise intravenös an einem Patienten / Probanden.

In einer bevorzugten Ausführungsform wird jedoch der erfindungsgemäße Kontrastverstärker iRGD in einer Dosis von 0,1 - 12 µmol/kg, 2 - 10 µmol/kg, vorzugsweise 4 µmol/kg Körpergewicht appliziert.

Die Applikation erfolgt vorzugsweise intravenös an einem Patienten / Probanden.

Die Kontrastmittel samt Kontrastverstärker der Erfindung können dabei sowohl alleine als auch in Kombination mit anderen diagnostischen, therapeutischen oder anderen Substanzen verwendet werden. Unter "anderen Substanzen" werden dabei insbesondere pharmazeutische Hilfsstoffe, Geschmacksstoffe oder Färbemittel verstanden. So können beispielsweise einer oral verabreichten Formulierung Saccharose oder ein natürliches Zitrusaroma beigemischt werden.

In einer weiteren Ausführungsform betrifft die Erfindung einen Kit umfassend eine oder mehrere Injektionslösungen zur Durchführung eines erfindungsgemäßen Verfahrens aufweisend unabhängig voneinander, gemeinsam oder jeweils ein Kontrastmittel, vorzugsweise Gd-DPTA und/oder einen Kontrastverstärker iRGD mit einer Dosis von 0,1 - 12 µmol/kg, 2 - 10 µmol/kg, vorzugsweise 4 µmol/kg Körpergewicht.

Beispielsweise enthält eine Injektionslösung 300 µmol iRGD für einen 75 kg Patienten. Gd-DPTA kann in üblicher Dosierung für eine MRT verabreicht werden.

Der Begriff "Risikostratifizierung" umfasst erfindungsgemäß das Auffinden von Patienten, insbesondere Notfallpatienten und Risikopatienten, mit der schlechteren Prognose, zwecks intensiverer Diagnostik und Therapie/Behandlung von Karzinomerkrankungen, insbesondere HCC mit dem Ziel einen möglichst günstigen Verlauf der Krankheit zu ermöglichen. Eine erfindungsgemäße Risikostratifizierung erlaubt in Folge ein effektives Behandlungsverfahren, die beispielsweise bei Karzinomerkrankungen mit den neueren Arzneimitteln, wie Zytostatika, monoklonalen Antikörpern und Chemotherapie gegeben sind bzw. zur Behandlung oder Therapie von Karzinomerkrankungen eingesetzt werden.

Daher betrifft die Erfindung ebenfalls die Identifizierung von Patienten mit erhöhtem Risiko oder/und einer ungünstigen Prognose von Karzinomerkrankungen und zwar bei symptomatischen und / oder asymptomatischen Patienten, insbesondere Notfallpatienten, z.B. aufgrund einer Metastasierung.

Besonders vorteilhaft kann insbesondere in Fällen der Notfall- und /oder Intensivmedizin mittels des erfindungsgemäßen Verfahren eine sichere Stratifizierung erfolgen. Das erfindungsgemäße Verfahren ermöglicht daher klinische Entscheidungen, die zu einem schnellen Therapieerfolg und zur Vermeidung von Todesfällen führen. Solche klinischen Entscheidungen umfassen ebenfalls weiterführende Behandlung mittels Arzneimitteln zur Behandlung oder Therapie von Karzinomen.

Daher betrifft die Erfindung ebenfalls ein Verfahren zur Diagnose und / oder Risikostratifizierung von Patienten von Karzinomerkrankungen zur Durchführung von klinischen Entscheidungen, wie weiterführende Behandlung und Therapie mittels Arzneimitteln, vorzugsweise in der zeitlich kritischen Intensivmedizin oder Notfallmedizin, einschließlich der Entscheidung zur Hospitalisierung des Patienten.

In einer weiteren bevorzugten Ausführungsform betrifft das erfindungsgemäße Verfahren daher die Therapiesteuerung von Karzinomerkrankungen bzw. Karzinomen.

In einer weiteren bevorzugten Ausführungsform des erfindungsgemäßen Verfahrens erfolgt die zur Diagnose und / oder Risikostratifizierung zur Prognose, zur differentialdiagnostischen Früherkennung und Erkennung, zur Beurteilung des Schweregrades und zur therapiebegleitenden Verlaufsbeurteilung. Die vorliegende Erfindung soll im Folgenden anhand von Beispielen und Figuren näher erläutert werden, ohne dass die Erfindung dadurch beschränkt wird.

### Beispiele und Figuren:

### 1. Peptide

Synthetische Peptide iRGD (CRGDKGPDC) und RGD Kontrollpeptid (CRGDDGPKC), welches in zirkulärer Form über eine Cystein-Cystein-Disulfidbindung zwischen AS 1 and 9 aufweisen (Sugahara et al., 2010), wurden von GenScript USA Inc. mit einer Reinheit von mehr als 98% erworben.

### 2. Erzeugung von TGFα/c-myc-transgenen Mäusen und Visualisierung der HCCs

Männliche TGFα/c-myc bitransgene Mäuse wurden durch die Kreuzung von homozygoten Metallothionein/TGFα und Albumin/c-myc einzel-transgenen Mäusen im CD13B6CBA Hintergrund wie beschrieben (Murakami et al., 1993; Haupenthal et al., 2012) erzeugt. Nach dem Abstillen erhielten die Tiere ZnCl₂ über das Trinkwasser zur Tumorinduktion über die Expression von TGFα. Ab einem Alter von 20 Wochen wurden die TGFα/c-myc Tiere einer Primovist-verstärkten MRT-Untersuchung in einem 3T MRI Scanner (Siemens 113 Magnetom Trio, Siemens Medical Solutions) wie beschrieben unterzogen (Haupenthal et al., 2012; ,Watcharin et al., 2015; Korkusuz et al., 2013).

Mäuse mit HCC gemäß Primovist-verstärkter MRT wurden in die nachfolgenden Experimente zugeführt.

### 3. Erzeugung der HCC-Nacktmäuse

HepG2- and Huh-7-Zellen (ATCC and RIKEN BioResource Center) wurden in DMEM mit 10% FBS und Penizillin/Streptomyzin (Life Technologies) kultiviert. 5 Millionen Zellen wurden in 100 µl PBS in die Flanken von NMRI Foxn1l Nacktmäuse (Harlan Laboratories B.V.) injiziert. Vier Wochen später wurden die Mäuse den Versuchsgruppen zugeordnet.

### 4. 4T1-Mamma-Karzinom-Mausmodell

4T1-Zellen (ATCC) wurden in RPMI 1640-Medium mit 10% FBS und Penizillin/Streptomyzin (Life Technologies) kultiviert. Balb/c-Mäuse wurden mit 2.5×104 4T1-Zellen in das die Mamma-Drüse Nr. 4 der Maus injiziert und die Tumore für zwei Wochen wachsen lassen.

### 5. Gd-DTPA-verstärktes MRT mit und ohne iRGD

Um die Wirkung von iRGD auf die Gd-DTPA-verstärkte MRT im HCC zu ermitteln, wurden TGFα/c-Mäuse mit HCC gemäß einer vorhergehenden Gd-EOB-DTPA (Primovist)-verstärkten MRT eine Woche vorher oder Nacktmäuse mit HepG2- oder Huh-7-Tumore in die Versuche eingeschlossen. Die Mäuse wurden mittels intraperitonealer Injektion von Ketamin (70 mg/kg Körpergewicht) und Xylazin (10 mg/kg KGt) anästhetisiert, gefolgt von einer basalen T1-gewichteten MRT. Direkt im Anschluss wurde ein Gd-DTPA-verstärktes MRT gefahren (Haupenthal et al., 2012). Zwölf bis 24 Stunden später wurde entweder iRGD oder RGD Kontrollpeptid (jeweils 100 ml über die Schwanzvene) injiziert, gefolgt von einem basalen und einem Gd-DTPA-verstärkten MRT (Watcharin et al., 2015). Zur quantitativen Analyse der MRT-Daten wurden die Signalintensität über benutzerdefinierte "regions of interest" (ROI) benutzt (Korkusuz et al., 2013). ROIs wurden in die Leber und in das Tumorgewebe gelegt. Die Veränderungen der Signalintensitäten wurden durch Subtraktion der Präkontrast-Werte von denjenigen nach Gd-DTPA-Gabe ermittelt. Die Veränderungen der Signalintensität der Tumore und der Lebern durch iRGD bzw. RGD-Kontrollpeptid wurden als Vielfaches der Werte des Gd-DTPA-MRTs mit vorheriger Injektion von PBS angegeben.

### Beschreibung der Ergebnisse:

Co-Administration von iRGD, jedoch nicht von einem Peptid, welches ein RGD-Motiv, nicht jedoch ein CendR-Motiv enthält, erhöht den Eintritt des Farbstoffes Evans blau und Doxorubicin selektiv in Karzinomen, insbesondere HCC.

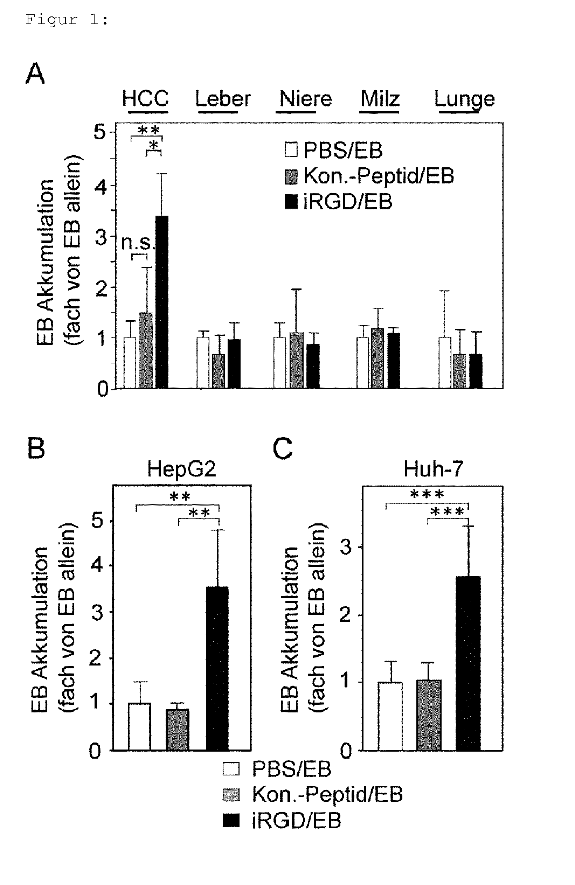

Um zu untersuchen, ob intravenös appliziertes iRGD die Permeabilität des HCC erhöht, wurde die Wirkung von intravenös appliziertem iRGD auf die Spiegel von co-injiziertem Evans blau, einem Albumin-bindende Farbstoff, in TGFα/c-myc Mäusen mit endogenen HCCs gemäß einem Gd-EOB-DTPA-verstärktem MRT, untersucht. Hierzu wurden die Mäuse mit HCC intravenös mit iRGD, einem RGD-Kontrollpeptid, welches kein CendR motif enthält, oder PBS injiziert, gefolgt von der Injektion von Evans blau 15 Minuten später. Weitere 30 Minuten später wurde die Tiere terminal perfundiert. Photometrische Quantifizierung des Farbstoffes ergab eine dreifach Erhöhung der Evans blau-Menge im Tumor von iRGD-injizierten Tieren im Vergleich zu HCCs von Mäusen, welche mit PBS- (p = 0,012) oder Kontroll-Peptid (p = 0,012) injiziert worden waren, während RGD-Kontroll-Peptid oder PBS keine Wirkung hatten (Figur 1A). iRGD oder das RGD-Kontrollpeptid hatte keine Wirkung auf die Konzentration von Evans blau in normalen Geweben (Leber, Niere, Milz und Lunge), was dafür spricht, dass iRGD spezifisch die Permeabilität des malignen Tumorgewebes der HCCs im TGFα/c-myc-Mäusen für co-applizierte Substanzen erhöht.

Um zu untersuchen, ob der Tumor-permeabilisierende Effekt von iRGD auch in HCC-Nacktmaustumormodellen auftritt, wurde die Wirkung von intravenös appliziertem iRGD auf die Spiegel von co-appliziertem Evans blau in Nacktmäusen untersucht, welche HepG2- oder Huh-7-Xenotransplantate trugen. iRGD bewirkte eine Erhöhung der Konzentrationen von co-appliziertem Evans blau in HepG2- sowie Huh-7-Xenotransplantierten Tumoren um den Faktor 3,4 (p ≤ 0.002) (in HepG2-Tumoren) bzw. 2,6-fach in Huh-7-Tumoren (p < 0.001) im Vergleich zu den entsprechenden Tumoren von PBS- oder Kontroll-Peptid-injizierten Nacktmusen (Figur 1B and C).

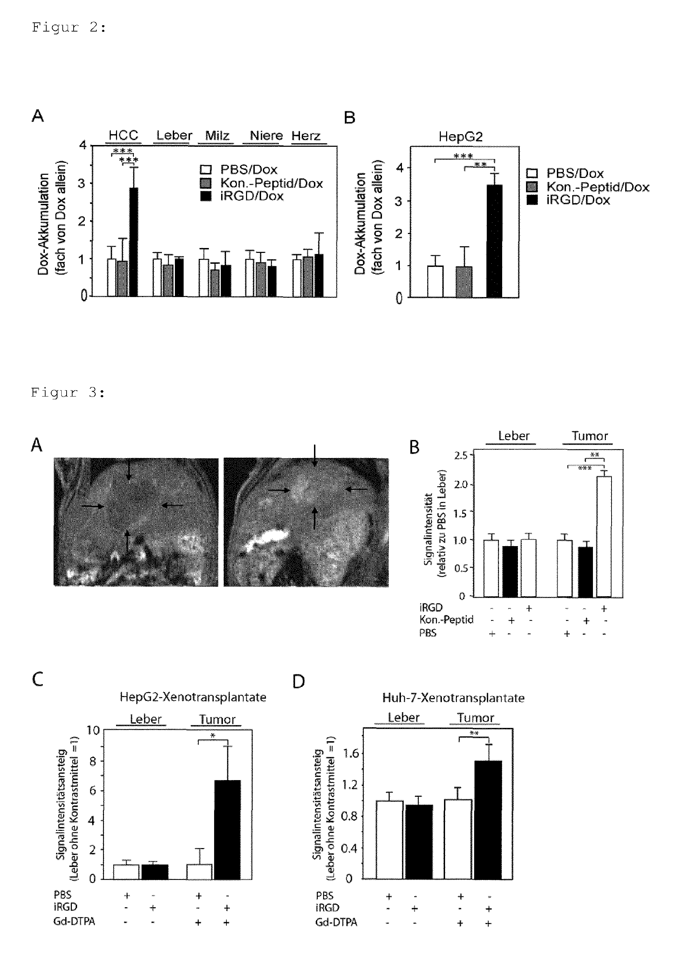

Weiterhin wurde die Wirkung von iRGD auf die Gewebekonzentrationen von intravenös appliziertem Doxorubicin, einem Tumor-Therapeutikum, welches anhand seiner Fluoreszenz in Gewebeschnitten und Gewebeextrakten detektiert werden kann, im TGFα/c-myc- und im HepG2-Xenotransplantat-HCC-Mausmodell untersucht. Wie in Figur 2 dargestellt ist, bewirkte iRGD eine Erhöhung der Doxorubicin-Spiegel in den HCCs in beiden Tumormodellen (p ≤ 0.0011). iRGD hatte keine Wirkungen auf die Spiegel von Doxorubicin in den Organen (Figur 2A). Das Kontroll-Peptid hatte keine Wirkungen auf die Doxorubicin in irgendeinem der Gewebe.

Es wurde untersucht, ob der Tumor-permeabilisierende Effekt von iRGD bereits durch ein Gd-DTPA-verstärkendes MRT, ein weit verbreitetes klinisches Verfahren, in TGFα/c-myc-Mäusen mit endogenen HCC detektiert werden kann. Dazu wurde TGFα/c-myc Mäuse mit eine Woche zuvor festgestellten HCCs gemäß Gd-EOB-DTPA-verstärktem MRT, verwendet. Diese Tiere erhielten ein Gd-DTPA-verstärktes MRT, welches stark negative kontrastierte HCC in der Leber zeigte (Figur 3B, links). 12 bis 24 Stunden später wurden den Mäusen iRGD oder RGD-Kontrollpeptid verabreicht, gefolgt von einem weiteren Gd-DTPA-verstärkten MRT. Wie in Figur 3A (links) gezeigt ist, blieben die Tumore von Mäusen, welchen PBS oder RGD-Kontrollpeptid injiziert worden war, negativ kontrastiert. Die Injektion von iRGD vor der Gd-DTPA-verstärkten MRT bewirkte eine substantielle Zunahme der Signalintensität der Tumore, welche nun positive in der Leber kontrastierten (Figur 3A, rechts). iRGD und RGD-Kontrollpeptid hatten keine Wirkungen auf die Signalintensitäten in normalen Organen. Die quantitative densitometrische Analyse von den Signalintensitäten zeigte eine 2-fache Zunahme in den Tumoren nach Injektion von iRGD im Vergleich zu Tieren, welchen RGD-Kontrollpeptid (p = 0,004) or PBS (p < 0,001; Figur 3B) verabreicht wurde.

Als nächstes wurde untersucht, ob iRGD auch die das MRT-Signal in HCCs in HCC-Xenotransplantat-Nacktmausmodellen beeinflußt. Wie in Figur 3C gezeigt ist, bewirkte iRGD einen Anstieg der Signalintensität der Tumore in HepG2-Tumoren (p = 0,0311). Ähnliches traf auf Huh-7-Xenotransplantate zu (p = 0,0081), aber der Anstieg war geringer ausgeprägt (1,5-fach; Figur 3D) im Vergleich zu den anderen HCC-Mausmodellen (≤ 2-fach). Diese Daten zeigen, dass der iRGD-induzierte Anstieg der Tumor-Permeabilität in allen drei verschiedenen HCC-Mausmodellen mittels dieses nicht-invasiven Verfahrens (Gd-DTPA-verstärktes MRT ohne und mit iRGD) beobachtet werden konnte.

Weiterhin wurde untersucht, inwieweit iRGD auch das MRT-Signal des Tumors im Gd-DTPA-verstärkten MRT im syngenen 4T1-Mamma-Karzinom-Mausmodell beeinflusst. Es zeigte sich, dass iRGD zu einem signifikanten Anstieg des MRT-Signals im Gd-DTPA-verstärkten MRT im 4T1-Tumor führte (p<0.05).

Daher erlaubt der Kontrastverstärker iRGD die Differentialdiagnostik einer gutartigen Veränderung von einer zirrhotisch veränderten Leber zu unterscheiden. Gegenwärtig wird zur radiologischen Diagnostik des HCC die unterschiedliche Blutversorgung von Leber und Tumorgewebe vorwiegend über die Pfortader (Leber) bzw. A. hepatica (HCC), was zu einer zeitlich verzögerten Anflutung von intravenös appliziertem Kontrastmittel in der Leber im Vergleich zum HCC-Gewebe führt, sowie die unterschiedliche Verteilung von Anionentransportern zwischen HCC und normalem Leberwebe. Letztere wird durch die Verteilung des Leber-spezifischen Kontrastmittels Primovist im MRT erreicht.
Figur 1:
   iRGD, jedoch nicht mit Kontroll-Peptid ohne CendR-Motiv, erhöhte die Konzentration von systemisch co-appliziertem Evans Blue (EB)-in den HCCs in TGFα/c-myc-Mäusen als auch in 2 HCC-Xenotransplantat-Nacktmausmodellen.
   TGFα/c-myc-Mäuse mit MRT-gesicherten HCCs (A) oder Mäuse mit subkutanen HepG2 (B) oder Huh-7-Xenotransplanten (C) wurden intravenös mit 4 mmol/kg iRGD oder Kontroll-Peptid (jeweils in PBS), oder PBS allein injiziert, gefolgt von einer followed Injektion von der Injektion von EB 5 Minuten später. Die Gewebe wurden nach weiteren 30 Minuten geerntet. Die EB-Akkumulation in den Geweben wurde photometrisch bestimmt. Die Daten sind Mittelwerte ± SD; n = 4-5. Sterne indizieren einen signifikanten Unterschied (* P < 0,05; * P < 0,01; *** P < 0,001). n.s., nicht signifikant.
Figur 2:
   Co-Behandlung mit iRGD erhöhte die Konzentrationen von Doxorubicin selektiv in HCCs von TGFα/c-myc-Mäusen und Mäusen mit HepG2-Xenotransplantaten.
   TGFα/c-myc-Mäuse mit radiologisch verifizierten HCCs (A) oder Nacktmäuse mit subkutanen HepG2-Xenotransplantaten (B) wurden intravenös mit 4 mmol/kg iRGD oder Kontroll-Peptide (jeweils in PBS), oder mit PBS allein behandelt, gefolgt von einer Doxorubicin (20 mg/kg i. v.)-Injektion 10 Minuten später. Die Gewebe wurden weitere 30 Minuten später asserviert und die Doxorubicin-Konzentration bestimmt. Die Werte sind Mittelwerter ± SD; n = 5-6. Sterne indizieren eine signifikante Differenz (**P < 0,01; ***P < 0,001).
Figur 3:
   iRGD führte zu einem Tumor-spezifischen Anstieg der Signalintensität in der Gd-DTPA-verstärkten MRT. TGFα/c-myc-Mäuse, in welchen HCCs eine Woche zuvor mittels Gd-EOB-DTPAverstärkter MRT nachgewiesen worden waren, wurden anästhetisiert, gefolgt von einer basalen T1-gewichteten MRT und direkt im Anschluß einer Gd-DTPA-verstärkten MRT. 12-24 Stunden später wurden entweder iRGD oder RGD-Kontrollpeptid (Kon.-Peptid) (jeweils 100 µl über die Schwanzvene, 4 mmol/kg)
   in dieselben Tiere injiziert, gefolgt von einer basalen und einer Gd-DTPA-verstärkten MRT. A, Gd-DTPA-verstärktes MRT von TGFα/c-myc-Mäusen mit HCC (links), gefolgt von einer Injektion von iRGD und einer weiteren Gd-DTPA-verstärkten MRT 12 Stunden danach. Pfeile markieren den Tumor. B, quantitative Analyse der MRT Daten. Werte sind Mittelwerte ±SD; n = 12.
   C, 12 Nacktmäuse mit HepG2-Xenotransplantaten wurden am Tag 1 mit Kontroll-Peptid injiziert, gefolgt einer Gd-DTPA-verstärkten MRT. Am nächsten Tag wurden die Tiere mit iRGD behandelt, gefolgt von einer Gd-DTPA-verstärkten MRT. Die Werte sind Mittelwerte ± SEM. D, 13 Nacktmäuse mit Huh-7-Xenotransplantaten wurden derselben Prozedur wie in (C) unterzogen. Die Werte sind Mittelwerte ± SEM. Sterne zeigen eine signifikante Differenz and (*P < 0,05; **P < 0,01; ***P < 0,001) .
Figur 4:
   Wirkung von iRGD und RGD-Kontrollpeptid auf den Anstieg der Signalintensität in der Gd-DTPA-verstärkten MRT in der Leber und im 4T1-Tumore in Balb/c-Mäusen. Balb/c-Mäuse wurden mit 2.5×10⁴ 4T1-Zellen in die Mamma-Drüse Nr. 4 der Maus injiziert. Zwei Wochen später wurden die Mäuse anästhetisiert, gefolgt von einer basalen T1-gewichteten MRT und direkt im Anschluss einer Gd-DTPA-verstärkten MRT. 12-24 Stunden später wurden entweder iRGD oder RGD-Kontrollpeptide (Kon.-Peptid) (jeweils 100 µl über die Schwanzvene, 4 mmol/kg) in dieselben Tiere injiziert, gefolgt von einer basalen und einer Gd-DTPA-verstärkten MRT. Die Daten wurden quantitativ analysiert. Werte sind Mittelwerte ±SD; n = 6.

### Literatur:

Sugahara KN, Teesalu T, Karmali PP, Kotamraju VR, Agemy L, Girard OM, et al. Tissue-penetrating delivery of compounds and nanoparticles into tumors. Cancer Cell 2009;16:510-20.
Sugahara KN, Teesalu T, Karmali PP, Kotamraju VR, Agemy L, Greenwald DR, et al. Coadministration of a tumor-penetrating peptide enhances the efficacy of cancer drugs. Science 2010;328:1031-5.
Teesalu T, Sugahara KN, Ruoslahti E. Tumor-penetrating peptides. Front Oncol 2013;3:216.
Murakami H, Sanderson ND, Nagy P, Marino PA, Merlino G, Thorgeirsson SS. Transgenic mouse model for synergistic effects of nuclear oncogenes and growth factors in tumorigenesis: interaction of c-myc and transforming growth factor a in hepatic oncogenesis. Cancer Res 1993;53:1719-23.
Haupenthal J, Bihrer V, Korkusuz H, Kollmar O, Schmithals C, Kriener S, et al. Reduced efficacy of the Plk1 inhibitor BI 2536 on the progression of hepatocellular carcinoma due to low intratumoral drug levels. Neoplasia 2012;14:410-9.
Watcharin W, Schmithals C, Pleli T, Köberle V, Korkusuz H,Hübner F, et al. Detection of hepatocellular carcinoma in transgenic mice by Gd-DTPA- and rhodamine-conjugated human serum albumin nanoparticles in T1 magnetic resonance imaging. J Control Release 2015;199:63-71.
Korkusuz H, Ulbrich K, Welzel K, Koeberle V, Watcharin W, Bahr U, et al. Transferrin-coated gadolinium nanoparticles as MRI contrast agent. Mol Imaging Biol 2013;15:148-54.
Ruoslahti E. Specialization of tumour vasculature. Nat Rev Cancer 2002;2:83-99.

## Patentansprüche

1. Bildgebendes Verfahren zur Diagnose und / oder Risikostratifizierung von Karzinomerkrankungen, **dadurch gekennzeichnet, dass** mittels
a.) eines Kontrastmittels enthaltend eine freie Lanthanoidverbindung, und
b.) eines Kontrastverstärkers iRGD,
c.) eine Magnetresonanztomographie erfolgt.

2. Bildgebendes Verfahren zur Diagnose und / oder Risikostratifizierung von Karzinomerkrankungen nach Anspruch 1, **dadurch gekennzeichnet, dass** mittels
a.) eines Kontrastmittels enthaltend eine freie Lanthanoidverbindung oder a'.) ein Kontrastverstärker iRGD,
b.) eine erste Magnetresonanztomographie erfolgt und zeitversetzt
c.) ein Kontrastmittel enthaltend eine freie Lanthanoidverbindung, und
d.) ein Kontrastverstärker iRGD,
e.) eine zweite Magnetresonanztomographie erfolgt.

3. Bildgebendes Verfahren zur Diagnose und / oder Risikostratifizierung von Karzinomerkrankungen nach Anspruch 2, wobei die zweite Magnetresonanztomographie innerhalb von 10 min. bis 6 h, insbesondere 1 bis 24 h, insbesondere innerhalb mehrerer Tage, insbesondere innerhalb von 4 bis 24 h nach dem ersten MRT erfolgt.

4. Bildgebendes Verfahren zur Diagnose und / oder Risikostratifizierung von Karzinomerkrankungen nach einem der vorherigen Ansprüche, **dadurch gekennzeichnet, dass** das Karzinom aus der Gruppe solide Malignome, Mamma-Karzinom, Kolon-Karzinom, Pankreaskarziom, Magenkarzinom, Ovarial-Karzinom, Gallenwegs-Karzinom, Prostata-Karzinom, Zervix-Karzinom, Glioblastom, Bronchial-Karzinom, Pankreaskarzinom, Prostata-Karzinom, Nierenzellkarzinom, Blasenkarzinom, Sarkome, hepato-zelluläres Karzinom (HCC), Hirntumore oder auch Tumormetastasen dieser Karzinome davon ausgewählt ist.

5. Bildgebendes Verfahren zur Diagnose und / oder Risikostratifizierung von Karzinomerkrankungen nach einem der vorherigen Ansprüche, **dadurch gekennzeichnet, dass** das iRGD aus der Gruppe mit den Sequenzen CRGDKGPDC (SEQ ID No.1), CRGDRGPDC (SEQ ID No.2), CRGDKGPEC (SEQ ID No.3), CRDGRGPEC (SEQ ID No.4) ausgewählt ist.

6. Bildgebendes Verfahren zur Diagnose und / oder Risikostratifizierung von Karzinomerkrankungen nach einem der vorherigen Ansprüche, **dadurch gekennzeichnet, dass** die freie Lanthanoidverbidnung eine Gadoliniumverbindung ist, insbesondere ein Komplex aus Gadolinum und einem Chelator ist, insbesondere wobei der Chelator ausgesucht ist aus der Gruppe bestehend aus Diethylentriaminpentaessigsäure (DTPA) und 1,4,7,10-Tetraazacyclododecan-1,4,7,10-Tetraessigsäure (DOTA).

7. Verfahren zur Diagnose und / oder Risikostratifizierung nach einem der vorherigen Ansprüche, zur Identifizierung von Patienten mit erhöhtem Risiko oder/und einer ungünstigen Prognose von Karzinomerkrankungen.

8. Verfahren zur Diagnose und / oder Risikostratifizierung nach einem der vorherigen Ansprüche, wobei der Patient ein symptomatischer und / oder asymptomatischer Patient, insbesondere ein Notfallpatient ist.

9. Verfahren zur Diagnose und / oder Risikostratifizierung nach einem der vorherigen Ansprüche, zur Therapiesteuerung von Karzinomerkrankungen, insbesondere in der Intensivmedizin oder Notfallmedizin.

10. Verfahren zur Diagnose und / oder Risikostratifizierung von Karzinomerkrankungen nach einem der vorherigen Ansprüche zur Durchführung von klinischen Entscheidungen, insbesondere weiterführende Behandlungen und Therapien mittels Arzneimitteln, insbesondere in der Intensivmedizin oder Notfallmedizin, einschließlich der Entscheidung zur Hospitalisierung des Patienten.

11. Verfahren zur Diagnose und / oder Risikostratifizierung von Karzinomerkrankungen nach einem der vorherigen Ansprüche zur Prognose, zur differentialdiagnostischen Früherkennung und Erkennung, zur Beurteilung des Schweregrades und zur therapiebegleitenden Verlaufsbeurteilung.

12. Kontrastmittel enthaltend a.) ein freies Lanthanoid und b.) ein Kontrastverstärker iRGD zur Verwendung in einem bildgebenden Verfahren zur Diagnose und / oder Risikostratifizierung von Karzinomerkrankungen, wobei eine Magnetresonanztomographie erfolgt.

13. Kontrastmittel enthaltend a.) ein freies Lanthanoid und b.) ein Kontrastverstärker iRGD zur Verwendung in einem bildgebenden Verfahren zur Diagnose und / oder Risikostratifizierung von Karzinomerkrankungen nach Anspruch 12, wobei mittels
a.) eines Kontrastmittels enthaltend eine freie Lanthanoidverbindung oder a'.) ein Kontrastverstärker iRGD,
b.) eine erste Magnetresonanztomographie erfolgt und zeitversetzt
c.) ein Kontrastmittel enthaltend eine freie Lanthanoidverbindung, und
d.) ein Kontrastverstärker iRGD,
e.) eine zweite Magnetresonanztomographie erfolgt.

14. Kontrastmittel enthaltend a.) ein freies Lanthanoid und b.) ein Kontrastverstärker iRGD zur Verwendung in einem bildgebenden Verfahren zur Diagnose und / oder Risikostratifizierung von Karzinomerkrankungen nach Anspruch 12, wobei iRGD intravenös appliziert wird.

15. Kit umfassend eine oder mehrere Injektionslösungen zur Durchführung eines Verfahrens nach einem der Patentansprüche 1 bis 13 aufweisend unabhängig voneinander, gemeinsam oder jeweils ein Kontrastmittel, insbesondere Gd-DPTA und/oder einen Kontrastverstärker iRGD mit einer Dosis von 0,1 - 12 µmol/kg, 2 - 10 µmol/kg, insbesondere 4 µmol/kg Körpergewicht.
